# EUROPEAN PATENT SPECIFICATION

(11) **EP 2 094 859 B1**
(45) Date of publication and mention of the grant of the patent: **24.11.2010**
(21) Application number: 07825981.9
(22) Date of filing: 03.08.2007
(51) Int. Cl.: C12P 19/04

(54) **PROCESS FOR PRODUCING A CELLULOSE-BASED FILM TO BE USED FOR SKIN AND TISSUE LESIONS**
VERFAHREN ZUR HERSTELLUNG EINES FÜR HAUT- UND GEWEBELÄSIONEN ZU VERWENDENDEN FILMS AUF CELLULOSEBASIS
PROCÉDÉ DE PRODUCTION DE FILM À BASE DE CELLULOSE DESTINÉ À ÊTRE UTILISÉ POUR DES LÉSIONS CUTANÉES ET TISSULAIRES

(30) Priority: 29.12.2006 IT TO20060932
(43) Date of publication of application: 02.09.2009
(73) Proprietor: BTC S.R.L., 60131 Ancona (AN) (IT)
(72) Inventor: MERIZZI, Gianfranco, I-10128 Torino (IT)
(74) Representative: Rambelli, Paolo
(86) International application number: PCT/IB2007/053063
(87) International publication number: WO 2008/081349

(56) References cited:
- WO-A-2005/003366
- TONOUCHI ET AL: "Increased cellulose production from sucrose by Acetobacter after introducing the sucrose phosphorylase gene" BIOSCIENCE, BIOTECHNOLOGY AND BIOCHEMISTRY, vol. 62, 1998, pages 1778-1780, XP002471356
- KIM ET AL: "Modification of Acetobacter xylinum bacterial cellulose using dextransucrase and alternansucrase" JOURNAL OF MICROBIOLOGY AND BIOTECHNOLOGY, vol. 9, 1999, pages 704-708, XP008006601
- CZAJA ET AL: "Microbial cellulose - the natural power to heal wounds" BIOMATERIALS, vol. 27, January 2006 (2006-01), pages 145-151, XP005071158

## Description

The present invention refers to a biotechnology process for producing a cellulose-based film, to be used for covering and regenerating skin and tissue lesions, and to a biosynthetic film thus obtained.

In particular, the invention refers to a process in which the aforesaid cellulose-based film is obtained by means of *Acetobacter xylinum* and Leuconostoc bacteria.

EP-A-0 114 481 describes the production of liquid-impregnated pads, useful as medication for wounds and bums, prepared from cellulose film produced microbially by means of *Acetobacter xylinum* culture. The process provides for the cultivation of the microorganism in static conditions (non-stirred culture); the bacteria are thus cultivated on the surface of the nutrient medium to form a consistent film, which generally has a 0.1 mm to approximately 15 mm thickness; the film thus obtained is removed from the nutrient medium, treated with sodium hydroxide to remove the bacteria, neutralised and washed with water to give a water-impregnated film of microbial cellulose.

WO86/02095 also describes the preparation of cellulose films, obtained from the cultivation of *Acetobacter xylinum,* used as artificial skin graft; a *Acetobacter xylinum* culture is inoculated in a culture medium containing nitrogen and carbohydrates as nutrients, medium in the specific examples being composed of an infusion of Tea Sinensis with added sugar; the process comprises the incubation of the microorganism at 28°C for a period of about 36 hours, after which the film is removed from the culture medium and dehydrated at room temperature on supports, in distended state. This patent underlies a medication commercialised with the BIOFILL mark, useful in the treatment of various skin lesions.

US 5 846 213 describes the preparation of cellulose-based medications having flexibility and mechanical properties similar to those of human skin. A cellulose film is prepared in advance by means of cultivation of *Acetobacter xylinum* and it is then dissolved in a solvent system comprising dimethylacetamide and a lithium salt so to obtain a solution from which a cellulose membrane is obtained by means of casting and coagulation in a gelling bath.

Tonouchi et al. (Biosci. Biotecnol. Biochem, 62(9), 1778-1780, 1998), discloses increasing cellulase production from *Acetobacter* cultures by transforming into the *Acetobacter* genome an expression plasmid containing a *Leuconostoc mesenteroides* gene coding for the enzyme sucrose phosphorilase under the control of the *lac* promoter.

Kim et al. (J. Microbiol-Biotecnol- 9(6) 704-708, 1999) discloses the modification of bacterial cellulase obtained from *Acetobacter* cultures by the activity of two enzymes, namely dextransucrase and alternansucrase, extracted from *Leuconostoc mesenteorides.*

The object of the present invention is that of providing a bacteria-produced cellulose film, and a process for its production, which is usable as medication for skin lesions and is effective in creating a favourable environment for the care of the lesion.

Another object is to provide a cellulose film which has suitable mechanical characteristics, in particular high flexibility, similar to the mechanical properties of the human skin.

Forming the object of the invention, therefore, are a process and a crystalline cellulose-based film having the characteristics defined in the following claims.

In particular, the process according to the invention is characterised in that the biosynthetic cellulose-based film is obtained by means of cultivation, in a culture medium containing nitrogen and carbon sources, of microorganisms belonging to the *Acetobacter xylinum* species, in the presence of at least one microorganism belonging to the *Leuconostoc* genus.

In a preferred embodiment, the process is conducted by inoculating a yeast in the culture medium as well, yeast preferably chosen from among *Saccharomyces cerevisiae, Schizosaccharomyces pombae* and/or *Saccharomyces malidevorans* and mixtures of two or three of these.

*Acetobacter* is a gram-negative, rod-shaped bacterium, strictly aerobic. It is characterised for the capacity to produce multiple chains of poly-beta-1,4-glucan, chemically identical to cellulose. The cellulose chains in microfibril form are synthesised on the bacterial surface at sites outside the cell membrane. In the scope of the invention, any strain of *Acetobacter xylinum* is used which is capable of producing cellulose microfibrils, both in static culture and in stirred culture conditions, including the specific strains described in EP 0 228 779.

The genus *Leuconostoc* comprises bacteria of spherical form, distinguishable from the genus *Lactococcus* by the production of gas. All of the species of the genus *Leuconostoc* have a heterofermentative metabolism; the bacteria are widely diffused in nature, especially on substrates of vegetal origin. They have optimal growth temperatures in the range of 20°C and 30°C and are less acidophilic than the lactobacilli, preferring weakly acidic or neutral substrates, except regarding *Leuconostoc oenos* (recently reclassified as *Oenoscoccus oenos*)*,* which is adapted to the low pH of wine and which is known as malolactic fermentation agent of wines.

Based on the tests carried out by the applicant, it is deemed that the presence of bacteria of the genus *Leuconostoc,* in the *Acetobacter xylinum* culture, considerably influences the production and growth of the biomass.

Film obtained by means of the process according to the invention is essentially composed of crystalline cellulose and is characterised by a relatively low average degree of polymerisation, generally in the range of 250 and 400. An important aspect of such film which characterises it with respect to classic crystalline celluloses is its insolubility in concentrated sulphuric acid. This aspect suggests that there are bonds between the single cellulose chains which make the product insoluble in concentrated sulphuric acid and difficult to hydrolyse.

In the process according to the invention, the cultivation of the abovementioned microorganisms is generally conducted at a temperature in the range of 20°C and 36°C and at a pH in the range of 1 and 6, preferably at a temperature from 26°C to 30°C and with pH in the range of 2 and 4.

The preferred culture medium for the symbiotic growth is constituted by cane sugar and/or by a liquid extract of cane sugar, as sources of carbon and nitrogen. The culture medium can moreover comprise sugar and acetic acid.

The culture is generally maintained in static conditions.

The film which forms is separated from the culture medium by means of *per se* known techniques and is dehydrated by drying.

When the film is rehydrated with physiological solution, it takes on characteristics of translucidity, flexibility and density similar to those of integral human skin; it is moreover equipped with selective permeability towards water vapour and gas and impermeability for water and bacteria. The film possesses an excellent adhesion capacity to the injured part; the occlusion which is generated protects the wound from the outside, preventing contamination, diminishing the pain and creating an ideal microenvironment for granulation and re-epithelialisation.

Such film is particularly recommended on burns, ulcers and sores of level I up to deep level II, without excess exudation; the film adheres perfectly to the injured part and is inert, non-toxic, non-allergenic and lacks latex.

### Example 1

In a 500 L stainless steel container, the following are inserted: 250 kg of cane sugar extract, 20 kg of cane sugar and vinegar in a quantity such to bring the mixture pH to 3.5. There is then the inoculation of the culture medium at room temperature with *Acetobacter xylinum* and *Leuconostoc* microorganisms, particularly *Leuconostoc oenos,* in 30 ml overall quantity. During this process, the pH is brought to and maintained at 2.5 by means of the addition of cane sugar and vinegar. Upon completion, the biomass is washed and refined up to the obtainment of the liquid state by means of the use of an industrial pulveriser; the liquid thus obtained is poured on a cotton support and housed in stainless steel cases connected in series with each other and then to a vacuum system which, by drying the liquid part, determines the formation of a smooth and uniform film on the cotton support.

The cotton support with adhered film is subsequently placed in the oven and once the drying process is complete, the film is removed from the support and sent to storage. Depending on the quantity of liquid biomass and on the size of the used case, films can be obtained of different size and thickness: from 750 ml of biomass in the liquid stated placed in a case of 23x30 cm size, for example, a film of 21x27 cm size arises with an approximately 0.05 mm thickness.

### Example 2

The procedure of example 1 is repeated at the same abovementioned conditions, using as inoculant, in addition to the abovementioned bacteria, also *Saccharomyces cerevisiae* and *Schizosaccharomyces pombae.*

The film obtained according to example 2 was subjected to analysis to verify the structure and determine the cellulose content.

The following analyses were conducted.
i) Recording the FT-IR spectrum (ASI React IR 1000 instrumentation with diamond cell - analysis of the sample as is - resolution of 4 cm⁻¹).

The recorded IR absorption signals are:
- 3340 cm⁻¹ OH stretching
- 2984 cm⁻¹ CH aliphatic stretching
- 1460-1200 cm⁻¹ CH₂ deformation, CH and CH₂ wagging and OH deformation in plane
- 1100-1000 cm⁻¹ CH₂O and CoC stretching
- 897 and 663 cm⁻¹ bands typical of cellulose.

The FT-IR spectrum therefore corresponds to that of cellulose.
ii) Analysis of the total carbohydrates by means of the HPLC method equipped with refraction index detector.

The methods used were those which are normally used to determine the cellulose and hemicellulose content.

The cellulose content was determined according to an internal method of the Stazione Sperimentale Carta Cartoni e Pasta per Carta (SSCCPC; Paper, Cardboard and Pulp Experimental Station) of Milan with total hydrolysis of the polysaccharides in concentrated sulphuric acid, followed by a measurement of the total reducing sugars (Somogyi-Nelson method) and determination of the glucose by enzymatic method.

The hemicellulose content was determined according to the SSCCPC internal method of selective hydrolysis of the hemicellulose in diluted sulphuric acid, followed by a measurement of the total reducing sugars (Somogyi-Nelson method).

The obtained results (expressed as percentage by weight with respect to the dry weight of the analysed sample) indicate that the obtained film is not soluble in the adopted conditions, conditions which usually lead to the hydrolysis of cellulose and hemicellulose.

The results obtained on the sample were the following:
- 1.7% of resinous substances extractible in cyclohexane/ethanol,
- 75% of residue insoluble after hydrolysis in concentrated (72%) sulphuric acid, followed by hydrolysis with diluted sulphuric acid (3%), or in experimental conditions which usually permit hydrolysing both the cellulose and hemicellulose into corresponding monosaccharides,
- 4.6% total reducing sugars determined on the solute of the hydrolysis,
- 1.8% of glucose (ascribable to cellulose),
- 2.8% total reducing sugars (ascribable to hemicellulose),
- 85% of residue insoluble after hydrolysis in 1 M sulphuric acid of the 75% of hydrolysis residue with 72% and 3% sulphuric acid (experimental conditions which usually permit selectively hydrolysing the hemicellulose to the corresponding monosaccharides),
- 2.5% total reducing sugars in the residue after hydrolysis in 1 M sulphuric acid (ascribable to hemicellulose).

In order to better define the real chemical structure of the film, an analysis was carried out through nuclear magnetic resonance at the solid state. The spectrum of the nucleus ¹³C was recorded by means of the DD CP-MAS (Dipolar Decoupling Cross-Polarization Magic Angle Spinning) technique.

This type of technique is necessary for reducing the dipolar couplings, and therefore for diminishing the signal bandwidth, a decoupling at high power and the rotation of the sample around an axis which is displaced with respect to the fixed axis by an angle of 54° 7', called the Magic Angle. The latter expedient allows eliminating the dipolar and homonuclear interactions and the chemical shift anisotropy. In particular, the DD CP-MAS technique was employed to increase the intensity of the signals of the diluted nuclei and to eliminate the problems connected with the overly long relaxation times of these same nuclei.

In this experiment, the spin polarisation of the abundant nuclei is utilised, such as for example the protons, which is relatively strong so to increase the spin polarisation of diluted nuclei, such as for example ¹³C.

The measurement was carried out with the As x 300 Bruker instrument, equipped with a 4 mm probe, by making the sample rotate at 8 kHz speed.

As results from the NMR spectra, the spectral profile has the typical signals of the glycosidic repetitive unit of the cellulose, whose profile results analogous to that of a cellulose I obtained from *Acetobacter.*

Moreover, from the whole crystalline C₄ and amorphous C₄, it is obtained that the crystalline percentage is about 75%, thus a sufficient value to be judged a high crystallinity index; in a further series of tests, crystallinity percentages were determined in the range of 65% and 90%.

The average degree of polymerisation was measured with the viscosimetric method UNI 8282/94. The sample was completely soluble in the used solvent (cupriethylenediamine), with average degree of polymerisation of 290.

## Claims

1. Process for producing a cellulose film, to be used for covering, regenerating, repairing and healing of skin and tissue lesions, such as sores, ulcers, bums, wounds and the like by means of cultivation, in a culture medium comprising nitrogen and carbon sources, of microorganisms comprising the *Acetobacter xylinum* species, **characterised in that** said culture medium moreover comprises at least one microorganism belonging to the *Leuconostoc* genus.

2. Process according to claim 1, **characterised in that** said culture medium moreover comprises a yeast chosen from among *Saccharomyces cerevisiae, Schizosaccharomyces pombae* and/or *Saccharomyces malidevorans.*

3. Process according to claims 1 or 2, **characterised in that** the cultivation is conducted at a temperature in the range of 20°C and 36°C and a pH in the range of 1 and 6.

4. Process according to any one of the claims 1 to 3, **characterised in that** the cultivation is carried out at a temperature in the range of 26°C and 30°C and a pH in the range of 2 and 4.

5. Process according to any one of the preceding claims, **characterised in that** the culture medium comprises cane sugar and/or liquid extract of cane sugar.

6. Process according to any one of the preceding claims, **characterised in that** the culture medium comprises acetic acid as pH corrector.

7. Process according to any one of the preceding claims, **characterised in that** as microorganism of the *Leuconostoc* genus, *Leuconostoc oenos* is used.

8. Crystalline cellulose-based biological film, used for skin and tissue covering, regenerating, repairing and healing, **characterised in that** it comprises crystalline cellulose in microfibril form having an average degree of polymerisation in the range of 250 and 400.

9. Biological film according to claim 8, **characterised in that** such film is insoluble in concentrated sulphuric acid.

10. Biological film according to claims 8 or 9, **characterised in that** it has a degree of crystallinity in the range of 65% and 90%.

11. Biological film according to any one of the claims 8 to 10, **characterised in that** said film is obtainable by means of a process according to any one of the claims 1 to 7.

## Patentansprüche

1. Verfahren zur Herstellung eines Cellulosefilms, der für Bedecken, Regenerieren, Reparieren und Heilen von Haut- und Gewebeläsionen, wie zum Beispiel Wunden, Geschwüren, Verbrennungen, Wunden und dergleichen verwendet werden soll mittels Kultivierung, in einem Kulturmedium umfassend Stickstoff- und Kohlenstoffquellen, von Mikroorganismen umfassend die *Acetobacter xylinum* Spezies, **dadurch gekennzeichnet, dass** das Kulturmedium darüber hinaus mindestens einen Mikroorganismus umfasst, der zur Gattung *Leuconostoc* gehört.

2. Verfahren nach Anspruch 1, **dadurch gekennzeichnet, dass** das Kulturmedium darüber hinaus eine Hefe ausgewählt aus *Saccharomyces cerevisiae, Schizosaccharomyces pombae* und/oder *Saccharomyces malidevorans* umfasst.

3. Verfahren nach Ansprüchen 1 oder 2, **dadurch gekennzeichnet, dass** die Kultivierung bei einer Temperatur im Bereich von 20°C und 36°C und einem pH-Wert im Bereich von 1 und 6 durchgeführt wird.

4. Verfahren nach einem der Ansprüche 1 bis 3, **dadurch gekennzeichnet, dass** die Kultivierung bei einer Temperatur im Bereich von 26°C und 30°C und einem pH-Wert im Bereich von 2 und 4 durchgeführt wird.

5. Verfahren nach einem der vorherigen Ansprüche, **dadurch gekennzeichnet, dass** das Kulturmedium Rohrzucker und/oder einen flüssigen Extrakt von Rohrzucker umfasst.

6. Verfahren nach einem der vorherigen Ansprüche, **dadurch gekennzeichnet, dass** das Kulturmedium Essigsäure als pH-Wert Korrektor umfasst.

7. Verfahren nach einem der vorherigen Ansprüche, **dadurch gekennzeichnet, dass** als Mikroorganismus der Leuconostoc-Gattung, *Leuconostoc oenos* verwendet wird.

8. Kristalliner auf Cellulose basierender biologischer Film, verwendet für Bedecken, Regenerieren, Reparieren und Heilen von Haut und Gewebe, **dadurch gekennzeichnet, dass** er kristalline Cellulose in Mikrofibrillen-Form mit einem mittleren Polymerisationsgrad im Bereich von 250 und 400 umfasst.

9. Biologischer Film nach Anspruch 8, **dadurch gekennzeichnet, dass** dieser Film unlöslich in konzentrierter Schwefelsäure ist.

10. Biologischer Film nach Ansprüchen 8 oder 9, **dadurch gekennzeichnet, dass** er einen Kristallinitätsgrad im Bereich von 65% und 90% hat.

11. Biologischer Film nach einem der Ansprüche 8 bis 10, **dadurch gekennzeichnet, dass** der Film mittels eines Verfahrens nach einem der Ansprüche 1 bis 7 erhältlich ist.

## Revendications

1. Procédé pour la production d'un film de cellulose, destiné à être utilisé pour couvrir, régénérer, réparer et guérir des lésions de la peau et de tissus, telles que des plaies, des ulcères, des brûlures, des blessures et lésions similaires par culture, dans un milieu de culture comprenant des sources d'azote et de carbone, de micro-organismes comprenant l'espèce *Acetobacter xylinum,* **caractérisé en ce que** ledit milieu de culture comprend en outre au moins un micro-organisme appartenant au genre *Leuconostoc.*

2. Procédé suivant la revendication 1, **caractérisé en ce que** ledit milieu de culture comprend en outre une levure choisie entre *Saccharomyces cerevisiae, Schizosaccharomyces pombae* et/ou *Saccharomyces malidevorans.*

3. Procédé suivant la revendication 1 ou 2, **caractérisé en ce que** la culture est effectuée à une température comprise dans l'intervalle de 20°C à 36°C et à un pH compris dans la plage de 1 à 6.

4. Procédé suivant l'une quelconque des revendications 1 à 3, **caractérisé en ce que** la culture est effectuée à une température comprise dans l'intervalle de 26°C à 30°C et à un pH compris dans la plage de 2 à 4.

5. Procédé suivant l'une quelconque des revendications précédentes, **caractérisé en ce que** le milieu de culture comprend du sucre de canne et/ou un extrait liquide de sucre de canne.

6. Procédé suivant l'une quelconque des revendications précédentes, **caractérisé en ce que** le milieu de culture comprend de l'acide acétique comme correcteur de pH.

7. Procédé suivant l'une quelconque des revendications précédentes, **caractérisé en ce que**, comme micro-organisme du genre *Leuconostoc, Leuconostoc oenos* est utilisé.

8. Film biologique à base de cellulose cristalline, utilisé pour couvrir, régénérer, réparer et guérir la peau et des tissus, **caractérisé en ce qu'**il comprend de la cellulose cristalline sous forme de microfibrilles ayant un degré moyen de polymérisation compris dans l'intervalle de 250 à 400.

9. Film biologique suivant la revendication 8, **caractérisé en ce qu'**il est insoluble dans l'acide sulfurique concentré.

10. Film biologique suivant la revendication 8 ou 9, **caractérisé en ce qu'**il a un degré de cristallinité compris dans l'intervalle de 65 % à 90 %.

11. Film biologique suivant l'une quelconque des revendications 8 à 10, **caractérisé en ce qu'**il peut être obtenu par un procédé suivant l'une quelconque des revendications 1 à 7.
